# EUROPEAN PATENT APPLICATION

(11) **EP 0 640 343 A1**
(43) Date of publication of application: **01.03.1995**
(21) Application number: 93305189.8
(22) Date of filing: 01.07.1993
(51) Int. Cl.: A61K 31/57

(54) **Contraceptive for oral use containing oestradial valerate and cyproterone acetate**

(71) Applicant: LEIRAS OY, SF-20210 Turku (FI)
(72) Inventor: Allonen, Hannu, SF-21610 Kirjala (FI); Posti, Juhani, SF-20320 Turku (FI)
(74) Representative: Allam, Peter Clerk

(57) **Abstract**

The present invention concerns an oral contraceptive containing, in combination, oestradiol valerate and cyproterone acetate, as well as at least one pharmaceutically acceptable carrier and optionally further adjuvants. The invention also concerns a method of contraception. According to a preferred embodiment, in a treatment cycle of 28 days, during a first period a) of 10 days a contraceptive is given containing 1 mg each of oestradiol valerate and cyproterone acetate, during a subsequent period b) of 11 days a contraceptive is given containing twice the amount of both said hormones. This period b) is followed by a 7 day period c) of no hormone administration.

## Description

The present invention relates to a combination contraceptive for oral use, to a method of contraception, as well as to a package assembly or package kit for use in the said method.

Development of oral contraceptives containing natural estrogens for women over 35, which are no longer in need of a very potent method of contraception, is most desirable. Such women are no longer very fertile, approximately only 60 % of women at the age of 40 having the capability of becoming pregnant, the number decreasing to 20 % at the age of 45. Still such women need some form of protection and preferably in a form without the adverse side-effects usually connected with combination oral contraceptives. It is generally accepted that the ethinyl oestradiol component used in current oral contraceptives is the major cause of thromboembolism, whereas progestogens are associated with arterial cardiovascular complications. Both problems tend to increase with age. Thus there is a clear need for a non-invasive contraceptive.

Natural 17β-oestradiol (E2) is widely used in post-menopausal replacement therapy without any serious side effects, although its use in oral contraceptives has not been successful because of high incidents of irregular bleeding. Of the 17α-hydroxyprogesteron derivatives available, only cyproterone acetate is widely used in oral contraceptives.

According to the invention an oral contraceptive has now been developed which is especially suited for women over the age of 35 up to menopause, the contraceptive containing, in combination, a natural 17β-oestradiol in the form of oestradiol valerate, and cyproterone acetate, as well as at least one pharmaceutically acceptable carrier and optionally further adjuvants.

Preferably the preparation contains equal amounts of oestradiol valerate and cyproterone acetate.

The invention also concerns a method of contraception comprising administering to a female subject in need thereof, an oral contraceptive preparation containing, in combination, oestradiol valerate and cyproterone acetate, according to the following regime
- administering daily during a first period a) of a cycle of 28 days, a preparation containing an effective amount, preferably 1 - 2 mg each of oestradiol valerate and cyproterone acetate, subsequently
- administering daily during a second period b) of the said cycle, a preparation corresponding to that administered during the first period a), or a preparation containing different, but effective amounts of oestradiol valerate and cyproterone acetate compared to those administered during the period a),
- the said periods a) and b) being followed by a third period c) of the cycle wherein no oestradiol valerate nor cyproterone acetate is administered.

Preferably during the period b) a preparation is used containing a higher amount, preferably twice the amount of both oestradiol valerate and cyproterone acetate compared to the amounts given during the period a). Also preferably during the period a), as well as during the period b), oestradiol valerate and cyproterone acetate are given in equal amounts.

During the period a), oestradiol valerate and cyproterone acetate are preferably each given in an amount of 1 mg.

During the second period b), the dose of each hormone is preferably raised to 2 mg.

In the treatment cycle of 28 days, the period a) is suitably 10 days, the period b) 11 days and the hormone free period 7 days. The treatment cycle is preferably started on the 3rd day counting from the first day of menstrual bleeding.

The invention also contemplates a kit for use in the method of contraception defined above, the kit containing
- first unit dosage forms of a preparation each containing an effective amount, preferably 1 - 2 mg each of oestradiol valerate and cyproteron acetate, together with at least one pharmaceutically acceptable carrier and optionally further adjuvants, for the administration of one of said first dosage forms daily during a first period a) of a cycle of 28 days,
- second unit dosage forms of a preparation each containing the same amounts of each of oestradiol valerate and cyproterone acetate as in the first unit dosage forms, or a preparation containing different, but effective amounts of oestradiol valerate and cyproterone acetate compared to those of the first dosage forms, together with at least one pharmaceutically acceptable carrier and optionally further adjuvants, for the administration of one of said second dosage forms daily during a second period b) of the said cycle,
- carrier means for the said unit dosage forms including a support, means for retaining the first unit dosage forms in in a first set of unit dosage forms, as well as for retaining the second unit dosage forms in a second set of unit dosage forms, and
- calender means attachable to the carrier means and arrangable to indicate a day or date associated with the corresponding unit dose to be administered that day during the periods a) and b).

Preferably the unit dosage forms for use during the periods a) and b) each contain equal amounts of oestradiol valerate and cyproterone acetate. During the period a) unit dosage forms containing 1 mg each of oestradiol valerate and cyproterone acetate are preferably used.

Preferably the unit dosage forms to be administered during the period b) contain a higher, preferably twice the amount of each of oestradiol valerate and cyproterone acetate compared to those in the first unit dosage forms. Thus the second unit dosage forms preferably contain each 2 mg of each of oestradiol valerate and cyproterone acetate.

The oral contraceptive according to the invention can be of any shape and form, representative examples being a tablet, granule, pellet, capsule or the like. The preparation according to the invention may, in addition to the active agents, contain conventional additives, such as carriers, diluents, fillers, lubricants, disintegrating agents etc. These are well known in the art.

The kit may suitably take the form of a so-called bubble package wherein each of the first and second unit dosage forms are enclosed in a separate bubble or blister on a support of e.g. cardboard, plastic or metal foil, to be released by simply compressing the blister and thus breaking the support at the bubble. The first unit dosage forms, of which there are preferably 10 per kit, are suitably arranged in a successive order or row to form a first set of unit dosage forms, and the second unit dosage forms, of which there preferably are 11 per kit, are similarly arranged in a successive order or row to form their own second set of unit dosage forms. The first and second unit dosage forms can suitably be aligned substantially along the circumference of a circle or square. The calender means are arranged to be attachable to the support so that the indivudual week days or alternatively dates indicated on the calender means are arranged to coincide with a respective unit dose to be adminstered that day during the periods a) and b).

According to a preferred embodiment, the unit dosage form of the first set are of a different colour from that of the unit dosage forms of the second set.

The oral contraceptive according to the invention has been studied in the following test:

### TEST REPORT

Fifty (50) healthy ovulating women aged between 35 and 47 yr using no other contraceptive precautions were randomly assigned on a double-blind basis to one of two groups treated with either formulation A, containing 1 mg of both oestradiol valerate and cyproterone acetate in the first 10 tablets and 2 mg of both in the following 11 tablets, or formulation B, containing equivalent doses of oestradiol valerate and norethisterone. These were used for 3-wk periods with a 1-wk no-treatment interval.

Fasting blood samples were drawn for hormone and lipoprotein determination. Blood samples for coagulation studies were taken from 16 women in Group A, twice during the pretreatment cycle and then at the end of the 1st and 3rd treatment cycles. For comparison purposes, blood coagulation parameters were also studied in 16 younger women who started to use a triphasic preparation (Trinordiol, Wyeth) containing ethinyl oestradiol and levo-norgestrel.

The following coagulation and fibrinolysis assays were performed: activated partial thromboplastin time (APTT), prothrombin-proconventin (P + P), fibrinogen, factors II, VII, VIII:C, vWf: Ag, VIII:Rcof, antithrombin III (antigen), protein C (antigen), plasminogen (functional) and alpha2-antiplasmin (antigen). The procedures have been described elsewhere [Laurell CB, Anal. Biochem 1966; 15: 45-52, Owren PA, J Clin Lab Invest 1949; 1: 81-83, Denson KW, Acta Haematol 1961; 25: 104-120, Lähteenmäki, P, Rasi V, Luukkainen T, Myllylä G., Am J. Obstet Gynecol 1981; 141: 175-179, Rasi V, Ikkala E, Torstila I, Thromb res 1982; 25: 203-212].

Serum lipoproteins were separated by means of PEG-6000 precipitation [Viikari J., Scand J Lab Invest 1976; 36: 265-268]. Total cholesterol and high-density-lipoprotein (HDL) cholesterol were determined by the ferric chloride method and triglycerides by the Boehringer Mannheim GmbH enzymatic method and colorimetric assay.

Serum sex-hormone-binding globulin (SHBG) was determined by an isotope-binding assay in the presence of cortisol [Hammond GL, Lähteenmäki P., Clin Chim Acta 1983; 132: 101-110]. Sex hormones were determined by radioimmunoassay. For the assays of progesterone, oestradiol (E2) and prolactin (PRL) iodinated tracers and other reagents produced by Farmos Diagnostica were used. E1 was determined after extraction using tritiated traces and an antiserum manufactured by Farmos Diagnostica. For the follicle-stimulating hormone (FSH) and luteinizing hormone (LH) asays Amersham International reagents were used.

In 22 patients (12 subjects in Group A and 10 in Group B) ovarian follicles, ovarian and uterine volumes were measured by ultrasound.

Endometrial specimens were taken from 7 subjects i each group before treatment and at the end of the 6th and 13th treatment cycles (using Vabra aspiration, Berkeley Medevices Inc. U.S.A.)

### RESULTS

The treatment groups did not differ with respect to body weight, height, parity or mean age. No changes in body weight or blood pressure were observed during treatment.

### - Hormone assays

The ovulatory serum FSH and LH peaks were suppressed in both groups, although the mid-luteal values were not affected. Serum PRL levels did not change. There were no difference between the mid-cycle and mid-luteal serum E2 values in the treatment cycles and the pretreatment cycle. As compared with the ovulatory and mid-luteal values in the pretreatment cycle, the serum E1 concentrations had doubled by day 14 of the treatment cycles, when the dose of E2 was 1 mg/day and quadrupled by day 22, when the dose of E2 was 2 mg/day.

As judged by the progesterone values, ovulation was inhibited in all subjects in Group A with the exception of one woman who ovulated during the first treatment cycle. In Group B ovulatory serum progesterone values were observed in 8 subjects, while in one woman all the treatment cycles were ovulatory and the treatment was therefore discontinued. In Group A the SHBG concentration was raised by 22% during the first three treatment cycles (*P* < 0.05), but the increase was not significant in later cycles.

### - Ultrasonic studies

The uterine volume measured by sonography before treatment was 146 ± S.E.M.). There were no significant changes during either of the treatment regimens.

### - Lipoproteins

Serum total cholesterol fell by 9% (*P* < 0.001) i Group A and 11% (*P* < 0.001) in Group B. The HDL-cholesterol concentration decreased by 12% in 3 mth in Group B (*P* < 0.001) and showed a tendency to decrease in group A also, although in this case the change was not significant until after 9 mth of treatment (*P* < 0.05). The atherogenic index (total cholesterol/HDL-cholesterol) improved in Group A (*P* < 0.01). No statistically-significant changes in triglyceride concentrations were observed.

### - Blood coagulation parameters

Except for the increase in plasminogen (*P* < 0.05) (Table III) , only minor changes were observed in eleven blood coagulation parameters studied in 16 subjects in Group A, whereas in a group of 16 younger women receiving an ordinary triphasic oval contraceptive significant increases in fibrinogen, plasminogen, alpha2-antiplasmin, P and P, protein C, factor vWf:Ag and factor VIII: Rcof were observed at the end of the third treatment cycle, together with a decrease in antithrombin III in the first treatment cycle.

### - Bleeding patterns

Eight (8) women i Group A and 7 in Group B had experienced profuse bleeding before entering the trial. In Group A, breakthrough bleeding was rare during treatment, although the incidence of spotting varied from 20% to 40%.

The amount of menstrual blood loss decreased in all subjects in Group A and the total number of bleeding days per cycle (including spotting and amenorrhoea) fell from 5.0 ± 1 days in the pretreatment cycle to 3.8 ± 3.3 days (mean ± S.D.) (*P* = 0.11) in the 12th treatment cycle.

In Group B the amount of blood loss decreased in 40% of the women and increased in 10%. Spotting was more frequent and 4 women discontinued the treatment because of breakthrough bleeding. The total number of bleeding days per cycle rose from 4.9 ± 1.2 days in th pretreatment cycle to 5.2 ± 2.5 days during treatment, although this change was not significant either.

Endometrial samples taken at the end of the 6th and 13th treatment cycles from 7 subjects in each group showed an almost ormal proliferative appearance. No endometrial hyperplasia was observed.

### - Side effects

Seven (7) subjects in Group A and 9 in Group B complained of premenstrual oedema, although this diminished after the first three treatment cycles.

The dysmenorrhoea reported by eight women in Group A disappeared during treatment. No pregnancies occurred. The degree of satisfaction at the end of the first year was quite high and 81% of the women in Group A and 67% i Group B were willing to continue the treament.

The results indicate that oestradiol valerate/cyproterone acetate combination was not found to have adverse effects on blood coagulation factors, whereas the triphasic low-oestrogen pill studied for comparision purposes had a clear effect on most of the blood coagulation parameters in the group of younger women.

The oestradiol valerate/cyproterone acetate combination studied meets the requirements for an oral contraceptive suitable for use by older women in many respects, since: (1) it exerts its action by inhibiting ovulation, which also protects against extrauterine pregnancy; (2) it prevents the development of endometrial hyperplasia and reduces heavy dysfunctional bleeding; (3) dysmenorrhoea disappears and premenstrual syndrome symptoms may decrease; and (4) it does not have adverse effects on blood coagulation or lipoprotein metabolism.

## Claims

1. Contraceptive for oral use containing, in combination, oestradiol valerate and cyproterone acetate, as well as at least one pharmaceutically acceptable carrier and optionally further adjuvants.

2. Contraceptive according to claim 1 containing equal amounts of oestradiol valerate and cyproterone acetate.

3. Contraceptive according to claim 2 containing 1 mg each of oestradiol valerate and cyproterone acetate per unit dosage form.

4. Contraceptive according to claim 2 containing 2 mg each of oestradiol valerate and cyproterone acetate per unit dosage form.

5. Method of contraception comprising administering to a female subject in need thereof, an oral contraceptive preparation containing, in combination, oestradiol valerate and cyproterone acetate, according to the following regime
- administering daily during a first period a) of a cycle of 28 days, a preparation containing an effective amount, preferably 1 - 2 mg each of oestradiol valerate and cyproterone acetate, subsequently
- administering daily during a second period b) of the said cycle, a preparation corresponding to that administered during the first period a), or a preparation containing different, but effective amounts of oestradiol valerate and cyproterone acetate compared to those administered during the period a),
- the said periods a) and b) being followed by a third period c) of the cycle wherein no oestradiol valerate nor cyproterone acetate is administered.

6. Method according to claim 5, wherein during the period b) a preparation is used containing a higher amount, preferably twice the amount of both oestradiol valerate and cyproterone acetate compared to the amounts given during the period a).

7. Method according to claim 6, wherein during the period a), as well as during the period b), oestradiol valerate and cyproterone acetate are given in equal amounts.

8. Method according to any one of claims 5 - 7, wherein in the treatment cycle, the period a) is 10 days, period b) is 11 days and the period c) is 7 days.

9. Method according to claim 8, wherein, during the period a), oestradiol valerate and cyproterone acetate are both administered daily in an amount of 1 mg.

10. Method according to claim 9, wherein, during the period a), oestradiol valerate and cyproterone acetate are both administered daily in an amount of 2 mg.

11. Kit for use in a oral method of contraception containing
- first unit dosage forms of a preparation each containing an effective amount, preferably 1 - 2 mg each of oestradiol valerate and cyproterone acetate, together with at least one pharmaceutically acceptable carrier and optionally further adjuvants, for the administration of one of said first dosage forms daily during a first period a) of a cycle of 28 days,
- second unit dosage forms of a preparation each containing the same amounts of each of oestradiol valerate and cyproterone acetate as in the first unit dosage forms, or a preparation containing different, but effective amounts of oestradiol valerate and cyproterone acetate compared to those of the first dosage forms, together with at least one pharmaceutically acceptable carrier and optionally further adjuvants, for the administration of one of said second dosage forms daily during a second period b) of the said cycle,
- carrier means for the said unit dosage forms including a support, means for retaining the first unit dosage forms in in a first set of unit dosage forms, as well as for retaining the second unit dosage forms in a second set of unit dosage forms, and
- calender means attachable to the carrier means and arrangable to indicate a day or date associated with the corresponding unit dose to be administered that day during the periods a) and b).

12. Kit according to claim 11, wherein the unit dosage forms for use during the periods a) and b) each contain equal amounts of oestradiol valerate and cyproterone acetate.

13. Kit according to claim 11 or 12, wheerin the unit dosage forms to be administered during the period b) contain a higher, preferably twice the amount of each of oestradiol valerate and cyproterone acetate compared to those included in the first unit dosage forms.

14. Kit according to claim 13, wherein the first set of unit dosage forms comprises 10 unit dosages, each containing 1 mg each of oestradiol valerate and cyproteron acetate, and the second set of dosage forms comprises 11 unit dosages, each containing 2 mg of oestradiol valerate and cyproteron acetate.

15. Kit according to any one of claims 11 - 14, wherein the said first unit dosage forms are of a different colour than that of the said second unit dosage forms.
